# EUROPEAN PATENT SPECIFICATION

(11) **EP 0 489 205 B1**
(45) Date of publication and mention of the grant of the patent: **13.03.1996**
(21) Application number: 90313156.3
(22) Date of filing: 04.12.1990
(51) Int. Cl.: A61F 13/15

(54) **An absorbent padding material**
Material für absorbierende Polsterung
Matériau pour un rembourrage absorbant

(43) Date of publication of application: 10.06.1992
(73) Proprietor: KANG NA HSIUNG ENTERPRISE CO. LTD., Tainan Hsien (TW)
(72) Inventor: Tai, Tsai-Sheng, Taipei (TW)
(74) Representative: Jones, Graham H.

(56) References cited:
- EP-A- 0 235 309
- EP-A- 0 336 578
- EP-A- 0 360 929
- WO-A-90/14061
- GB-A- 2 014 508

## Description

This invention relates to an absorbent padding material which may be used, for example, as a personal sanitary product.

Personal sanitary products such for example sanitary towels, sanitary sponges, nappies, diapers and diaper shorts are well known. These known personal sanitary products use absorbent padding material which is not entirely satisfactory. More specifically, the known absorbent padding material usually comprises a liquid-retaining material having a special liquid-absorbing contact surface. The liquid-retaining material is usually a high molecular padding material. The liquid-absorbing contact surface can be any one of several known types of structure. It is usually the liquid-absorbing contact surface which controls the operation of the absorbent padding material and the known liquid-absorbing contact surfaces suffer from disadvantages during use.

A first known liquid-absorbing contact surface is constructed as a polyethylene film on the liquid-retaining material. The polyethylene film has a plurality of apertures through which the liquid to be absorbed passes to the liquid-retaining material. The apertures are inwardly tapering apertures which taper towards the liquid-retaining material. These tapering apertures slow down the passage of liquid to be absorbed through the polyethylene film so that absorbent padding material with this type of liquid-absorbing contact surface takes a relatively long while to absorb liquid, and also does not quickly recover to a dry state. This type of liquid-absorbing contact surface is often known as a dry mesh film. The dry mesh film has a special degree of elasticity and special equipment is required to overcome this elasticity. Thus, in addition to having a relatively slow liquid-absorbing speed and a relatively slow recovery rate, absorbent padding material produced with the dry mesh film tends to require the use of special equipment which adds to manufacturing costs.

A second known liquid-absorbing contact surface is a polyethylene structure which has off-set apertures and which is provided on a non-woven cloth. The off-set apertures are parallel-sided apertures but they are in two separate layers in the polyethylene structure. The apertures in the polyethylene structure communicate with each other through side slots formed as the polyethylene structure is manufactured. This second type of liquid-absorbing contact surface is able to filter liquids faster than the above mentioned first type of liquid-absorbing contact surface. However, the speed of absorbing liquids is still not fast enough.

Generally, known sanitary products having a high molecular liquid-retaining material and a special liquid-absorbing contact surface never really attain the required aim of fast liquid absorption and the speedy return of the liquid-absorbing contact surface to a dry state. Once the liquid, mainly water, is absorbed by the high molecules, coagulated particles tend to form and these coagulated particles tend to prevent the satisfactory use of the known absorbent padding material. It is known to provide slots in the high molecular liquid-retaining material for the purpose of guiding the flow of liquid to be absorbed, but such slots only very slightly improve the absorption speed of the absorbent padding material.

It is also known from EP-A-0360929 to provide an absorbent padding material comprising a liquid-absorbing contact surface formed of a layer of plastics material provided on a layer of a non-woven cloth. The layer of the plastics material has a plurality of apertures through which the liquid to be absorbed passes to the non-woven cloth. These apertures are parallel sided bores which help to facilitate the speed through which the liquid to be absorbed is able to pass through the layer of the plastics material. In EP-A-0360929 the layer of the plastics material is described as a film which means that the layer of the plastics material is thin. Also in EP-A-0360929 the layer of the non-woven cloth is described as a web which means that the layer of the non-woven cloth is thick This agrees with what is shown in the drawings of EP-A-0360929 wherein the layer of the plastics material is shown as being substantially thinner than the layer of the non-woven cloth. This arrangement in which the layer of the plastics material is thinner than the layer of the non-woven cloth prevents the liquid-absorbing contact surface quickly being able to recover its liquid-absorbing properties after use.

It is an aim of the present invention to provide an absorbent padding material which is an improvement over the above mentioned known absorbent padding materials.

Accordingly, this invention provides an absorbent padding material comprising a liquid-absorbing contact surface formed of a layer of plastics material provided on a layer of a non-woven cloth, the layer of the plastics material being provided with a plurality of apertures through which the liquid to be absorbed passes to the non-woven cloth, and the apertures being parallel-sided through bores in combination with the layer of the plastics material being thicker than the layer of the non-woven cloth, for facilitating the speed with which the liquid to be absorbed is able to pass through the layer of the plastics material and for facilitating the speed with which the liquid-absorbing contact surface is able to recover its liquid-absorbing properties after use.

It will be appreciated from the above that the absorbent padding material of the present invention is advantageous in that the liquid to be absorbed is able to pass through the layer of the plastics material quickly, which facilitates the speedy absorption of liquids to be absorbed. Furthermore, because the liquid is able to pass quickly through the liquid-absorbing contact surface, the liquid-absorbing contact surface is able to recover its liquid-absorbing properties quickly after use by returning to its dry state. Still further, the liquid-absorbing contact surface is such that the layer of the plastics material can be provided without the need for special manufacturing machines of the type required to overcome the elasticity of the known absorbent padding material having the plastics material with the two layers of off-set apertures. Preferably, the layer of the plastics materials is 0.1mm thick.

Preferably, the apertures are circular apertures in plan view. The apertures may however have any suitable and appropriate shape in plan view, so long as they are parallel sided through bores.

The plastics material is preferably polyethylene. Other plastics materials may however be employed.

Preferably, the absorbent padding material is one in which the layer of the plastics material is so provided on the layer of the non-woven cloth that the plastics material and the cloth form an integral composite layer.

The integral composite layer preferably has a thickness of less than 0.15mm.

The absorbent padding material may be one in which the integral composite layer is formed by spreading the plastics material in molten form on to the non-woven cloth and thereafter passing the non-woven cloth and the hot plastics material through a machine for compressing and spreading the hot plastics materials into a required layer and for providing the apertures in the plastics material. The apertures may however be formed by means other than punching if desired.

The absorbent padding material will usually be manufactured to include liquid-retaining material. The liquid-retaining material will usually form the bulk of the absorbent padding material. The liquid-retaining material is preferably a high molecular padding material. Such a high molecular padding material may be one which is known in itself and which is used in known absorbent padding materials.

The present invention also extends to a personal sanitary product when including an absorbent padding material of the invention.

The personal sanitary products may be a sanitary towel, a sanitary sponge, a nappy, a diaper or diaper shorts. Other products may also be produced from the absorbent padding material.

An embodiment of the invention will now be described solely by way of example and with reference to the accompanying drawings in which:
Figure 1 is a cross section through part of a first known absorbent padding material;
Figure 2 is a cross section through part of a second known absorbent padding material; and
Figure 3 is a cross section through part of an absorbent padding material in accordance with the present invention.

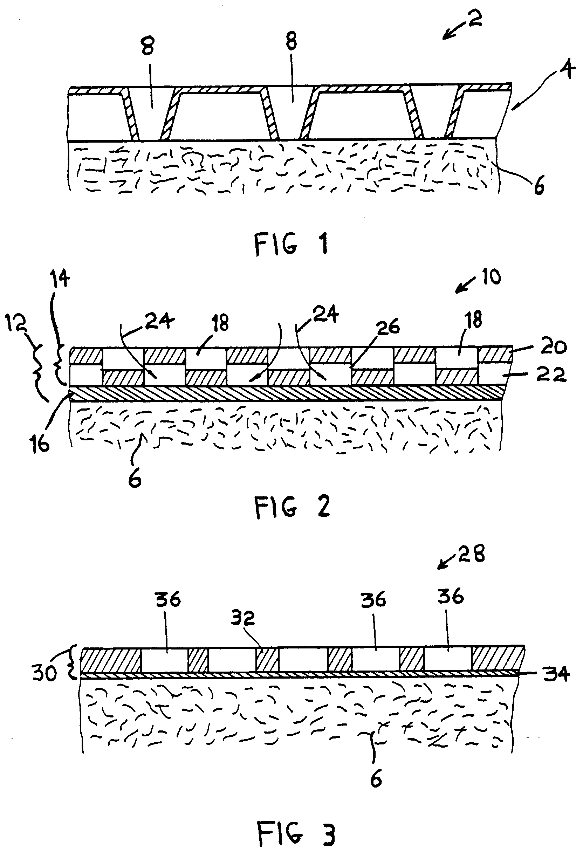

Referring to Figure 1, there is shown an absorbent padding material 2 comprising a liquid-absorbing contact surface 4. The liquid-absorbing contact surface 4 is a polyethylene film and the polyethylene film is provided on a liquid-retaining material in the form of a high molecular padding material 6. The liquid-absorbing contact surface 4 is provided with apertures 8 and these apertures 8 taper as shown towards the high molecular padding material 6. These tapering apertures 8 slow down the passage of liquid to be absorbed through the liquid-absorbing contact surface 4 so that the liquid to be absorbed cannot be absorbed quickly. Furthermore, the liquid-absorbing contact surface 4 does not quickly recover to its dry state. The liquid-absorbing contact surface 4 shown in Figure 1 is often known as a dry mesh film. This dry mesh film has a special degree of elasticity and special equipment is required to overcome this elasticity.

The slow absorption speed of the liquid-absorbing contact surface 4 shown in Figure 1 is set out in Table 1 hereinbelow. The slow passage of the liquid to be absorbed through the apertures 8 is attributed to the tapering sides of the aperture and the fact that the length of the tapering bores forming the apertures is at least 0.3mm, which is a relatively long length.

Referring now to Figure 2, there is shown a second known absorbent padding material 10. This absorbent padding material 10 comprises a liquid-absorbing contact surface 12 which is provided on high molecular padding material 6. The liquid-absorbing contact surface 12 comprises a polyethylene structure 14 provided on a non-woven cloth 16. The polyethylene structure 14 has off-set apertures 18 as shown. The off-set apertures 18 are parallel-sided apertures but they are in two separate layers 20,22 in the polyethylene structure 14 as shown. The off-set apertures 18 communicate with each other as shown by arrows 24, the communication being through side slots 26 formed as the polyethylene structure 14 is manufactured. As can be seen from the arrows 24, the liquid to be absorbed has to travel a relatively long distance through the polyethylene structure 14 and this liquid may have to travel longer than the 0.3mm distance mentioned above for the apertures 8 in the liquid-absorbing contact surface 4 illustrated in Figure 1. Because the off-set apertures 18 are parallel sided, it is believed that this helps to increase the speed of the passage of the liquid to be absorbed through the polyethylene structure 14 but, due to the relatively long distance required to be travelled by the liquid to be absorbed, the liquid-absorbing contact surface 12 still does not operate fast enough, even although it operates faster than the liquid-absorbing contact surface 4.

In Figure 2, the non-woven cloth 16 is provided on the illustrated side of the polyethylene structure 14 in order to prevent absorbed liquid from filtering back out of the high molecular padding material 6. The provision of the polyethylene structure 14 on the non-woven cloth 16 requires the use of modified manufacturing machinery since the polyethylene structure 14 is elastic and this elasticity must be overcome by the manufacturing machine.

Referring now to Figure 3, there is shown absorbent padding material 28 comprising a liquid-absorbing contact surface 30. The liquid-absorbing contact surface 30 is formed of a layer of a plastics material 32 which is provided on a layer of a non-woven cloth 34. The layer of the plastics material 30 is provided with a plurality of apertures 36 through which the liquid to be absorbed passes to the layer of the non-woven cloth 34. The apertures 36 are parallel-sided through bores as shown. These parallel-sided through bores facilitate the speed with which the liquid to be absorbed is able to pass through the layer of the plastics material 32, and they also facilitate the speed with which the liquid-absorbing contact surface 30 is able to recover its liquid-absorbing properties after use and to return to its dry state.

As can be seen from Figure 3, the layer of the non-woven cloth 34 is provided on high molecular padding material 6. As can also be seen from Figure 3, the layer of the plastics material 32 is thicker than the layer of the non-woven cloth 34. The layer of the plastics material 32 is 0.1mm thick. The apertures 36 are circular in plan view.

The plastics material is polyethylene and the layer of the plastics material 32 is so provided on the layer of the non-woven cloth 34 that the plastics material and the cloth form an integral composite layer. The integral composite layer has a thickness of less than 0.15mm. The integral composite layer is formed by spreading the plastics material in molton form on to the non-woven cloth and thereafter passing the non-woven cloth and the hot plastics material through a machine (not shown) for compressing and spreading the hot plastics material into the required layer of a plastics material 32 and for providing the apertures 36 in the plastics material. The apertures 36 are punched apertures 36 which are punched by the machine.

The absorbent padding material 28 shown in Figure 3 is advantageous as compared with the absorbent padding material 2 shown in Figure 1 and with the absorbent padding material 10 shown in Figure 2. More specifically, because the apertures 36 are parallel sided through bores which communicate directly with the layer of the non-woven cloth 34, the speed of absorption of a liquid to be absorbed is very fast. The layer of the non-woven cloth 34 helps to prevent liquid already absorbed by the high molecular padding material 6 from passing back through the apertures 36. Preferably, as shown, the layer of the non-woven cloth 34 is thinner than the layer of the non-woven cloth 16. The hot compressing and spreading of the plastics material to form the layer of the plastics material 32 increases the strength of the layer of the non-woven cloth 34. The absorbent padding material 28 shown in Figure 3 is able very quickly to return to its state of dryness so that it can be maintained substantially constantly dry.

Referring now to Table 1, there are shown the results from comparative tests in which A represents an absorbent padding material according to the present invention and of the general type shown in Figure 3, B represents an absorbent padding material of the type shown in Figure 2, and C represents an absorbent padding material of the type shown in Figure 1.

**TABLE 1**

| Absorbent padding material | A | B | C |
|---|---|---|---|
| Absorption speed (seconds) | 3.06 | 7.45 | 12.26 |
| Dryness (g) | 0.02 | 1.27 | 0.03 |

From Table 1, it can be seen that the absorbent padding material of the present invention as shown in Figure 3 has a faster absorption speed than the absorbent padding material shown in Figure 2. The absorbent padding material shown in Figure 2 has a faster absorption speed than the absorbent padding material shown in Figure 1. The speed of absorption of the absorbent padding material shown in Figure 3 is believed to be due to the fact that the liquid-absorbing contact surface 30 is very thin, and also because the liquid to be absorbed passes through apertures 36 which are through bores having vertical sides. With regard to dryness, it will be seen from Table 1 that the liquid-absorbing contact surface of the absorbent padding material shown in Figure 3 is the one which returns to a dry state the fastest. The liquid-absorbing contact surface shown in Figure 1 is the next fastest one to return to the dry state, and the liquid-absorbing contact surface shown in Figure 2 is the slowest one to return to the dry state. It is concluded from the comparative results shown in Table 1 that the liquid-absorbing contact surface 30 shown in Figure 3 will always exhibit the fastest filtration speed and dryness. This is believed to be so irrespective of the particular type of liquid-retaining material used in the absorbent padding material so that the liquid-retaining material may be the high molecular padding material 6 or other liquid-retaining material which is not of a high molecular type. The present invention thus provides a significant advantage over the absorbent padding materials currently available and as typified by the absorbent padding materials shown in Figures 1 and 2. Furthermore, the layer of the plastics material 32 may be in the form of a plastics film which can be provided, for example punched, with a variety of different sized and shaped parallel-sided apertures in order to suit the requirements of different types of absorbent padding materials.

It is to be appreciated that the embodiment of the invention described above with reference to the accompanying drawings has been given by way of example only and that modifications may be effected. Thus, for example, the apertures 36 shown in Figure 3 may be of a different shape to those shown which are circular in plan view. Furthermore, the high molecular padding material 6 may be replaced by another type of liquid-retaining material.

## Claims

1. An absorbent padding material (28) comprising padding (6) having a liquid-absorbing contact surface (30) formed of a layer of plastics material (32) provided on a layer of a non-woven cloth (34), the layer of the plastics material (32) being provided with a plurality of apertures (36) through which the liquid to be absorbed passes to the non-woven cloth (34), characterised by the combination of the apertures (36) being parallel-sided through bores and the layer of the plastics material (32) being thicker than the layer of the non-woven cloth (34), for facilitating the speed with which the liquid to be absorbed is able to pass through the layer of the plastics material (32), and for facilitating the speed with which the liquid-absorbing contact surface (30) is able to recover its liquid-absorbing properties after use.

2. An absorbent padding material according to claim 1 in which the layer of the plastics material (32) is 0.1mm thick.

3. An absorbent padding material according to claim 1 or claim 2 in which the apertures (36) are circular apertures in plan view.

4. An absorbent padding material according to any one of the preceding claims in which the layer of the plastics material (32) is so provided on the layer of the non-woven cloth (34) that the plastics material and the cloth form an integral composite layer.

5. An absorbent padding material according to claim 4 in which the integral composite layer has a thickness of less than 0.15mm.

6. An absorbent padding material according to claim 4 or claim 5 in which the integral composite layer is formed by spreading the plastics material in molten form on to the non-woven cloth and thereafter passing the non-woven cloth and the hot plastics material through a machine for compressing and spreading the hot plastics material into the required layer and for providing the apertures (36) in the plastics material.

7. An absorbent padding material according to any one of the preceding claims and including a liquid-retaining material (6).

8. A personal sanitary product when comprising an absorbent padding material (28) according to any one of the preceding claims.

## Patentansprüche

1. Absorbierendes Polstermaterial (28) mit einem Polster (6), das eine flüssigkeitsabsorbierende Kontaktoberfläche (30) aus einer auf eine Faservliesschicht (34) aufgebrachten Schicht aus Kunststoffmaterial (32) mit einer Mehrzahl Öffnungen (36) aufweist, durch die hindurch die zu absorbierende Flüssigkeit zu der Faservliesschicht (34) gelangt, ***gekennzeichnet*** durch die Kombination folgender Markmale:
Die Öffnungen (36) sind Durchbohrungen mit parallelen Wänden und die Kunststoffmaterialschicht (32) ist dicker als die Faservliesschicht (34), um die Geschwindigkeit, mit der die zu absorbierende Flüssigkeit durch die Kunststoffmaterialschicht (32) hindurchzutreten vermag wie auch die Geschwindigkeit zu vergrößern, mit der die flüssigkeitsabsorbierende Kontaktoberfläche (30) nach Gebrauch ihre flüssigkeitsabsorbierenden Eigenschaften wiedererlangt.

2. Absorbierendes Polstermaterial nach Anspruch 1, bei welchem die Kunststoffmaterialschicht (32) 0,1 mm dick ist.

3. Absorbierendes Polstermaterial nach Anspruch 1 oder 2, bei welchem die Öffnungen (36) in Draufsicht kreisförmig sind.

4. Absorbierendes Polstermaterial nach irgendeinem der vorhergehenden Ansprüche, bei welchem die Kunststoffmaterialschicht (32) derart auf die Faservliesschicht (34) aufgebracht ist, daß das Kunststoffmaterial und das Vlies eine einheitliche zusammengesetzte Schicht bilden.

5. Absorbierendes Polstermaterial nach Anspruch 4, bei welchem die einheitliche zusammengesetzte Schicht eine Dicke von weniger als 0,15 mm besitzt.

6. Absorbierendes Polstermaterial nach Anspruch 4 oder Anspruch 5, bei welchem die einheitliche zusammengesetzte Schicht durch Aufbringen des Kunststoffmaterials in geschmolzener Form auf das Faservlies und anschließendes Hindurchführen des Faservlieses und des heißen Kunststoffmaterials durch eine Maschine zum Komprimieren und Verteilen des heißen Kunststoffmaterials zu der gewünschten Schicht und zur Herstellung der Öffnungen (36) in dem Kunststoffmaterial hergestellt ist.

7. Absorbierendes Polstermaterial nach irgendeinem der vorhergehenden Ansprüche, das ein flüssigkeitszurückhaltendes Material (6) enthält.

8. Persönlichkeitsbezogenes Sanitärerzeugnis mit einem absorbierenden Polstermaterial (28) nach irgendeinem der vorhergehenden Ansprüche.

## Revendications

1. Matériau à rembourrage absorbant (28) comprenant un rembourrage (6) présentant une surface de contact absorbant les liquides (30) formée d'une couche de matière plastique (32) prévue sur une couche d'une étoffe non tissée (34), la couche de matière plastique (32) étant pourvue d'un grand nombre d'orifices (36) par lesquels le liquide à absorber passe à l'étoffe non tissée (34), caractérisé par la combinaison de ce que les orifices (36) sont des trous traversants, à cotés parallèles, et de ce que la couche de matière plastique (32) est plus épaisse que la couche d'étoffe non tissée (34), pour faciliter la vitesse à laquelle le liquide à absorber est capable de passer à travers la couche de matière plastique (32) et pour faciliter la vitesse à laquelle la surface de contact absorbant les liquides (30) est capable de récupérer ses propriétés d'absorption de liquide, après usage.

2. Matériau à rembourrage absorbant suivant la revendication 1, caractérisé en ce que la couche de matière plastique (32) a une épaisseur de 0,1 mm.

3. Matériau à rembourrage absorbant suivant l'une des revendications 1 et 2, caractérisé en ce que les orifices (36) sont des orifices circulaires dans une vue en plan.

4. Matériau à rembourrage absorbant, suivant l'une quelconque des revendications précédentes, caractérisé en ce que la couche de matière plastique (32) est prévue sur la couche d'étoffe non tissée (34) de façon que la matière plastique et l'étoffe forment une couche composite solidaire.

5. Matériau à rembourrage absorbant suivant la revendication 4, caractérisé en ce que la couche composite solidaire présente une épaisseur inférieure à 0,15 mm.

6. Matériau à rembourrage absorbant suivant l'une des revendications 4 et 5, caractérisé en ce que la couche composite solidaire est formée par étalement de la matière plastique sous forme fondue sur l'étoffe non tissée et ensuite par passage de l'étoffe non tissée et de la matière plastique chaude à travers une machine destinée à comprimer et étaler la matière plastique chaude en la couche requise et à réaliser les orifices (36) dans la matière plastique.

7. Matériau à rembourrage absorbant suivant l'une quelconque des revendications précédentes, comportant une matière de retenue de liquide (6).

8. Produit sanitaire personnel, comprenant un matériau à rembourrage absorbant (28) suivant l'une quelconque des revendications précédentes.
